Europäisches Patentamt

European Patent Office

Office européen des brevets

(19)

(11) EP 0 956 899 B1

(12) EUROPEAN PATENT SPECIFICATION

(45) Date of publication and mention
of the grant of the patent:
**05.02.2003 Bulletin 2003/06**

(51) Int Cl.7: **B01J 23/89**, C07C 5/333

(21) Application number: **96937974.2**

(86) International application number:
**PCT/CN96/00101**

(22) Date of filing: **14.11.1996**

(87) International publication number:
**WO 97/018034 (22.05.1997 Gazette 1997/22)**

(54) **A CATALYST FOR MANUFACTURING ALKYL AROMATICS AND PRODUCTION METHOD AND USE THEREOF**

KATALYSATOR FUR DIE HERSTELLUNG VON ALKYLAROMATEN UND VERFAHREN ZU DESSEN HERSTELLUNG UND DESSEN VERWENDUNG

CATALYSEUR POUR LA PRODUCTION D'ALKYLES AROMATIQUES, ET PROCEDE DE PRODUCTION ET UTILISATION DESDITS ALKYLES

(84) Designated Contracting States:
**DE**

(30) Priority: **15.11.1995 CN 95113340**

(43) Date of publication of application:
**17.11.1999 Bulletin 1999/46**

(73) Proprietors:
• **CHINA PETRO-CHEMICAL CORPORATION
Beijing 100029 (CN)**
• **Shanghai Research Institute of Petrochemical
Technology
Shanghai 201208 (CN)**

(72) Inventors:
• **MAO, Liansheng
Shanghai 201208 (CN)**
• **YUAN, Yiting
Shanghai 201208 (CN)**
• **FAN, Qin
Shanghai 201208 (CN)**
• **XU, Yongfan
Shanghai 201208 (CN)**
• **YANG, Cheng
Shanghai 201208 (CN)**

(74) Representative: **Goddar, Heinz J., Dr.
FORRESTER & BOEHMERT
Pettenkoferstrasse 20-22
80336 München (DE)**

(56) References cited:
EP-A- 0 389 255        CN-A- 1 005 019
CN-A- 1 006 686        CN-C- 1 021 637
CN-C- 1 028 495        GB-A- 1 464 252
US-A- 4 458 098        US-A- 5 023 225
US-A- 5 190 906

• CHEMICAL ABSTRACTS, vol. 118, no. 15, 12 April 1993 (1993-04-12) Columbus, Ohio, US; abstract no. 147285, MAO, LIANSHENG ET AL: "Catalysts for dehydrogenation of alkylarenes" XP002096618 -& CN 1 062 678 A (CHINA GENERAL PETROCHEMICAL CO., PEOP. REP. CHINA)

## Description

Technical Field

The invention relates to a catalyst for the dehydrogenation of alkyl aromatics and a process for the dehydrogenation of alkyl aromatics using said catalyst. More particularly, the catalyst of this invention can be used for producing styrene from ethylbenzene, divinylbenzene from diethylbenzene and methylstyrene from methylethylbenzene respectively by the dehydrogenation.

Technical Background

It is well known that, in industrial production, unsaturated aromatics are generally produced by the catalytic dehydrogenation of alkyl aromatics, however, one of the cruxes in said production process is to select a catalyst having high efficiency in dehydrogenation. It is reported from relative informations that catalysts can be divided into two main classes according to their main components: the first class of catalysts which comprises iron-potassium-chromium as main components with other elements added thereto, features a low water ratio of feeding (e.g. ratio of steam to ethylbenzene), inferior selectivity, generally less than 94%, and a low conversion, and lower total yield of styrene, for example, US Patent 4,533,650 has disclosed a catalyst system which contains iron-potassium-chromium as the main components with the addition of some rare-earth metal elements, but this system contains poisonous chromium component on the one hand, and gives lower total yield of styrene on the other hand, with the maximum yield being only up to 62.2%; the second class of catalysts contains iron-potassium-cerium-molybdenum as main components with the addition of other elements, for example, in the published Chinese Patent ZL 9 110 9968.9, it is described that the selectivity and conversion of this class of catalysts are higher than the first, but the total yield of styrene is only about 70%. Generally, in the industrial production, it requires the selectivity, conversion and yield of styrene to be high, the higher, the better.

In the dehydrogenation techniques for producing unsaturated aromatics, the dehydrogenation of ethylbenzene is the most typical technique and is of high economic value, so people have been concerned with the research in the catalysts for dehydrogenation of ethylbenzene. Half a century ago, the first generation of the catalyst for dehydrogenating ethylbenzene was developed, however, the conversion of ethylbenzene was only about 50% and the selectivity was about 90~92%. In 1980s, the properties of the catalysts for dehydrogenating ethylbenzene were further improved, with the conversion going up to 73% and the selectivity up to 94.5%. It is well known that the reaction of forming styrene by dehydrogenating ethylbenzene is an equilibrium reaction; while the reaction of forming styrene becomes stronger, its reverse reaction is carrying on simultaneously, hence it adds to the difficulties in increasing the yield of styrene. In the recent ten years, scientists have conducted go-in-depth researches in the catalysts for this kind of reaction, but little result has been achieved, while the selectivity and conversion of the catalysts still fluctuate at the original level. Futhermore, the reaction of forming styrene by dehydrogenation of ethylbenzene is a reaction of forming two molecules from one molecule, i.e., a volume-expanded reaction. In the past, in order to increase the reaction rate, steam was often added during the reaction to bring down the partial pressure of ethylbenzene, so that the yield would be increased. Steam plays the role of regenerating the catalyst at a high temperature, the volume of steam added is connected with the energy consumption in the plant. If a large volume of steam is added, more energy will be consumed in the plant, and in respect of the equipment, the production capacity will be reduced, therefore, we wish to find a desirable catalyst which is applicalte to a limited addition of steam, i.e., a low ratio of steam to alkyl aromatics (e.g. ethylbenzene) (known as a low water ratio) is required. If the properties of the dehydrogenation catalyst are improved, a lower water ratio will be required. When the water ratio is gradually decreased from the original value 3.0(weight ratio) to 1.5(weight ratio), for a catalyst having poor properties, the conversion and selectivity will reduce more rapidly under low water ratio, and the catalyst will even lose its activity, or hence be unregenerate.

As the industrial production of styrene by the dehydrogenation of ethylbenzene is in a very large scale, generally with an annual output of over ten thousand tons and even several hundred thousand tons, therefore, a little improvement in the properties of the dehydrogenation catalysts will result in big benefits to the enterprises. Even if the yield is increased only by one percentage point with the improved catalyst, for an industrial unit of the 10,000-ton class in scale, a yearly net increment of several hundred tons of the product will be attained without changing any equipment and increasing investment, meanwhile the operation efficiency will be raised and the consumption of materials and energy will be also reduced. Therefore, while seeking a catalyst suitable for operating at a low water ratio, further to improve the selectivity and conversion for the catalyst is a research topic concerned constantly by people.

Disclosure of the invention

An object of the present invention is to provide a dehydrogenation catalyst for the production of unsaturated aromatics, which comprises Fe-K-Mo-Mg-rare earth metal elements as main components, having improved conversion, selectivity and yield properties.

Another object of the present invention is to provide a process for producing unsaturated aromatics using the catalyst said above, which includes the dehydrogenation of alkyl aromatics to produce unsaturated aromatics.

Still another object of the invention is to provide a use of said catalyst in the production of unsaturated aromatics. Said catalyst can be used in producing styrene from ethylbenzene, divinylbenzene from diethylbenzene and also methylstyrene from methylethylbenzene by the dehydrogenation respectively.

Detailed description of the invention

The first object of the present invention is achieved in that said catalyst comprises $Fe_2O_3$ 40~70%, $K_2O$ 10~40%, $MoO_3$ 0~5%, MgO 0.05~5% and 0.001~5% of at least one oxide of the elements selected from the group consisting of Cu, Zn, Sc, Ti, W, Mn, Ni, Pd, Al, P, Bi, B, Sn, Pb and Si by weight, and that the catalyst comprises further at least two compounds of rare-earth metal elements, the content of which is from 2% to 15% on the basis of the oxides, and the rare-earth metal elements of the catalyst are selected from the group consisting of Ce, La, Pr, Nd and Sm.

It is preferred that the content of MgO is from 0.5% to 4% by weight.

In another embodiment of the invention, the content of the at least one oxide of the elements selected from the group consisting of Cu, Zn, Sc, Ti, W, Mn, Ni, Pd, Al, P, Bi, B, Sn, Pb and Si is from 0.002% to 2% by weight percent.

In a further embodiment of the invention, the content of the rare-earth metal elements in the catalyst is from about 3% to 8% based on the oxides.

Furthermore, it is preferred that one rare-earth metal element is cerium.

In one alternative embodiment of the invention, said catalyst comprises further a pore-forming agent and an adhesive.

The second object of the invention is achieved in that said unsaturated aromatics are produced by contacting alkyl aromatics with said catalyst under the conditions of dehydrogenation, and the catalyst used comprises: $Fe_2O_3$ 40~70%, $K_2O$ 10~40%, $MoO_3$ 0~5%, MgO 0.05~5% and 0.001~5% of at least one oxide of the elements selected from the group consisting of Cu, Zn, Sc, Ti, W, Mn, Ni, Pd, Al, P, Bi, B, Sn, Pb and Si by weight percent, and further comprises at least two compounds of rare-earth metal elements , the content of which is from 2% to 15% on the basis of the oxides, and the rare-earth metal elements of the catalyst are selected from the group consisting of Ce, La, Pr, Nd and Sm.

Preferably, the reaction condition of said dehydrogenation includes the reaction temperature from 550 to 650°C, the reaction pressure from atmosphere to -0.07 MPa, the weight ratio of water to alkyl aromatics from 0.6 to 2.5, and the liquid hourly space velocity from 0.3 to 1.0 hr$^{-1}$.

Most preferably, the reaction temperature is from 600°C to 630°.

In a most preferred embodiment, the reaction pressure is from -0.02 to -0.05 MPa.

Alternatively, the weight ratio of water/alkyl aromatics is from 1.0 to 1.5.

In a further embodiment of the invention, the liquid hourly space velocity is from 0.4 to 0.6 hr$^{-1}$.

In another embodiment of the invention, the content of the compounds of rare-earth metal elements in the catalyst is from about 3 % to 8 % based on the oxides.

In a preferred embodiment, the content of MgO by weight percent is from 0.5 % to 4 %.

In a most preferred embodiment, the content of at least one oxide of the elements selected from the group consisting of Cu, Zn, Sc, Ti, W, Mn, Ni, Pd, Al, P, Bi, B, Sn, Pb, and Si is from 0.002 % to 2 % by weight percent.

Furthermore, said catalyst comprises a pore-forming agent and an adhesive.

The third object of the present invention is achieved by a use of said catalyst according to the invention in the preparation of styrene, divinylbenzene or methylstyrene.

The dehydrogenation catalyst of the invention for producing unsaturated aromatics comprises (by weight percent): $Fe_2O_3$ 40~70%, $K_2O$ 10~40%, rare-earth metal oxide 2 ~15%, $MoO_3$ 0~5%, MgO 0.05~5% (by weight), wherein iron used is added in the form of iron oxide prepared by oxidating an acidic ferrous salts; wherein potassium used is added in the form of potassium salts; wherein magnesium used is added in the form of the oxides of magnesium, the content of which is preferably in the range of 0.5~4%; rare-earth metal elements used are selected from at least two of the group consisting of Ce, La, Pr, Nd and Sm, the content of which added is preferably in the range of 3~8 % based on the oxides; molybdenum used is added in the form of molybdenum salts or oxides, and also added are multiple metal oxides, which can be optionally selected at least one or more oxides of the elements from the group consisting of Cu, Zn, Sc, Ti, W, Mn, Ni, Pd, Al ,P, Bi, B, Sn, Pb and Si, the content of which is in the range of 0.001~5%, preferably in the range of 0.002-2%, the rest are a pore-forming agent and an adhesive, said pore-forming agent can be polystyrene microspheres, graphite or carboxymethyl cellulose; said adhesives can be selected from common adhesives, such as cement and etc..

In the invention, two or more rare-earth metal elements are used; with the addition of which in the catalyst, it is surprisingly found that this catalyst's product yield is increased by several percentage points over the original, while the

selectivity still remains at a higher level. It is appropriate that the content of rare-earth metal element added shall be in the range from 2% to 15%; if the content of rare-earth metal element is less than 2%, the dispersion degree will be insufficient to stabilize the components; if the amount added is over 15%, the distribution of rare-earth metal will be overdense, and then the conversion will be decreased.

In the catalyst of the invention, the combination of multiple elements is employed, wherein iron and potassium are the main active components, through electron transfer of the $Fe^{+2}$ and $Fe^{+3}$ under the cocatalysis of potassium, the catalyst will have higher activity. The elements of magnesium and rare-earth metal play the role of dispersing and stabilizing the components of the catalyst, and an adequate amount of molybdenum added will be advantageous to the selectivity of the catalyst. The addition of the elements of IB~VIIIB group and IIIA~VA group can bring down the reduction temperature of the catalytic reaction. Satisfactory results are obtained through the combination of various elements said above in accordance with the proportion of the invention.

Preparation method of the catalyst of the invention:

After being weighed according to the proportion of the invention, Fe, K, rare-earth metal elements, Mo, alkali-earth metal, element of B group and element of A group, an adhesive and a pore-forming agent are mixed homogeneously, then an appropriate amount of deionized water is added to form a dough body with stickiness and suitable to be extruded, then the final product of the catalyst is obtained by extruding and cutting into particles of 3 mm in diameter and 8~10 mm in length, and then by calcinating at 500 ~ 1000 °C for 3 ~ 10 hours.

Under a given process condition, the dehydrogenation catalyst of the present invention can be applicable for dehydrogenating respectively ethylbenzene, diethylbenzene and methylethylbenzene to form correspondingly styrene, divinylbenzene and methylstyrene.

The catalyst of the present invention provides higher activity and product yield than those dehydrogenation catalysts currently used in industry, and also has stronger ability of self-regeneration, so the regeneration is not necessary during normal operation. It is suitable for long running under a low water ratio such as 1.3 (the water ratio is less than 1.5), especially when the steam feeding is suddenly interrupted during the reaction, even for a short time (less than 20 minutes), the catalyst will not be deactivated, and will still maintain the original conversion and selectivity, manifesting the higher self-regenerating ability of the catalyst of the present invention.

The evaluation of the activity of the catalyst prepared according to the invention is carried out in an isothermal fixed-bed reactor; with regard to the evaluation of the activity of forming styrene by the dehydrogenation of ethybenzene, the procedures are described briefly as follows:

After deionized water and ethylbenzene are introduced respectively to a preheater-mixer with a metering pump, and preheated and mixed to gaseous state, the resultant mixture is fed into the reactor and heated to a predetermined temperature; the reactor is a stainless-steel tube having an inner diameter of 1 inch (about 25.4 mm), in which 100 ml of the catalyst particles of 3 mm in diameter can be packed; after the effluent reactant from the reactor is cooled and condensed with water, the composition of the product is analyzed by a gas chromatograph.

The conversion of ethylbenzene (hereinafter abbreviated to EB in the equation as following) and the selectivity of styrene (hereinafter abbreviated to SR only in the equation as following) is calculated according to the equation as follows:

$$\text{Conversion of EB\%} = (\text{EB content before reacting (wt\%)} - \text{EB content after reacting(wt\%)}) \div \text{EB content before reacting (wt\%);}$$

$$\text{Selectivity of SR} = (\text{SR content formed (wt\%)}) \div (\text{EB content before reacting (wt\%)} - \text{EB content after reacting(wt\%)}).$$

The present invention is described in detailed by following examples.

Example 1

In a kneader, 279 g of iron oxide, 54 g of potassium carbonate, 50 g of cerium oxalate, 10 g of neodymium oxalate, 9 g of magnesium oxide, 0.9 g of copper oxide, 35 g of cement and 4.1 g of carboxymethyl cellulose were stirred for 1~6 hours, then taken out and extruded to form particles of 3 mm in diameter and 8~10mm in length, then placed into a calciner, calcined at 600°C for 6 hours, then the catalyst was obtained. 100 ml of the catalyst particles of 3 mm in diameter were placed into the isothermal reactor having an inner diameter of 1 inch (about 25.4 mm) for evaluation of its activity under the reaction conditions of atmosphere pressure, 620°C, the liquid hourly space velocity of 1.0 (liter ethylbenzene/liter catalyst • hr) and 2.0 (weight ratio) of water ratio(water/ ethylbenzene), the evaluation results were: the conversion of ethylbenzene 78.20%; the selectivity of styrene 95.35% and the yield of styrene 74.5%.

Example 2

According to the method of the example 1, the catalyst was prepared by using 175 g of iron oxide, 97 g of potassium carbonate, 30 g of cerium oxalate(7.38%), 30 g of praseodymium oxalate, 5.5 g of ammonium molybdenate, 6 g of magnesium oxide, 0.5 g of cobalt nitrate, 15 g of cement and 6 g of carboxymethyl cellulose; and the evaluation was carried out in the same apparatus and under the same conditions as that of the example 1; the evaluation results: the conversion of ethylbenzene 79.40%; the selectivity of styrene 95.60% and the yield of styrene 75.90%.

Example 3

According to the method of the example 1, the catalyst was prepared by using 275 g of iron oxide, 80 g of potassium carbonate, 3 g of cerium molybdenate, 4 g of praseodymium molybdenate, 3 g of neodymium molybdenate, 8 g of magnesium oxide, 20 g of bismuth nitrate, 0.9 g of copper oxide, 22 g of cement and 8 g of carboxymethyl cellulose, and the activity of the catalyst was evaluated in the same apparatus and under the same conditions as that of with the example 1; the evaluation results: the conversion of ethylbenzene 79.69%, the selectivity of styrene 95.41% and the yield of styrene 76.03%.

Example 4

According to the method of the example 1, the catalyst was prepared by using 270 g of iron oxide, 72 g of cerium nitrate, 30 g of samarium nitrate, 10 g lanthanum nitrate, 16 g of ammonium molybdenate, 130 g of potassium carbonate, 8 g of magnesium oxide, 9 g of nickel oxide, 0.9 g of copper oxide, 0.1 g of boron oxide, 0.9 g of titanium oxide, 0.1 g of tin oxide, 0.1g of tungsten oxide, 22 g of cement and 9 g of carboxymethyl cellulose. The evaluation was carried out in the same apparatus and under the same conditions as that of the example 1, except for a reaction temperature of 600°C and the water ratio of 1.5(weight ratio), the evaluation results: the conversion of ethylbenzene 68.53%, the selectivity of styrene 95.76% and the yield of styrene 65.62%.

Example 5

According to the method of the example 1, the catalyst was prepared by using 270 g of iron oxide, 130 g of potassium carbonate, 8 g of ammonium molybdenate, 48 g of cerium nitrate, 50 g of praseodymium nitrate, 20 g of neodymium nitrate, 10 g of lanthanum nitrate, 5 g of magnesium oxide, 2.35 g of zinc oxide, 0.1 g of palladium oxide, 0.1 g of lead oxide, 22 g of cement and 9 g of carboxymethyl cellulose; and the evaluation was also carried out in the same apparatus and under the same conditions as that of the example 1, the evaluation results: the conversion of ethylbenzene 80.15%, the selectivity of styrene 96.08% and the yield of styrene 77.00%; when the water ratio was decreased to 1.3(weight ratio), the conversion of ethylbenzene was 76.82%, the selectivity of styrene 95.14% and the yield of styrene 73.08%.

Example 6

According to the catalyst composition of the example 4, using the evaluation conditions same as the example 1, the evaluation results: the conversion of ethylbenzene 79.68%; the selectivity of styrene 95.38% and the yield of styrene 75.99%. Then, still according to the apparatus and reaction conditions of the example 1, ethylbenzene was fed continuously, and the steam, after being interrupted for 20 minutes, was introduced again for reacting, the evaluation results were obtained: the conversion of ethylbenzene 79.46%, the selectivity of styrene 95.25% and the yield of styrene 75.68%, showing that the catalyst of the present invention has very strong ability of self-regeneration.

Example 7

According to various steps of the example 1, the catalyst was prepared by using 290 g of iron oxide, 60 g of potassium carbonate, 30 g of praseodymium oxalate, 8.5 g molybdenum oxide, 7 g of magnesium oxide, 0.9 g of copper oxide, 37 g of cement and 4.5 g of carboxymethyl cellulose, and the activity evaluation was carried out. The evaluation results: the conversion of ethylbenzene 72.8%, the selectivity of styrene 95.28% and the yield of styrene 69.36%.

Example 8

According to the method of the example 1, the catalyst was prepared by using 275 g of iron oxide, 70 g of potassium carbonate, 3.8 g of praseodymium molybdenate, 4.1 g of lanthanum nitrate, 6 g of magnesium oxide, 0.5 g of cobalt nitrate, 1.0 g of copper oxide, 25 g of cement and 7 g of carboxymethyl cellulose, and the activity evaluation was carried out according to the evaluation conditions of the example 1; the evaluation results: the conversion of ethylbenzene 76.65%; the selectivity of styrene 95.40% and the yield of styrene 73.12%.

Example 9

According to the method of the example 1, the catalyst was prepared by using 270 g of iron oxide, 130 g of potassium carbonate, 8 g of ammonium molybdenate, 48 g of cerium nitrate, 5 g of magnesium oxide, 2.35 g zinc oxide, 0.1 g of palladium oxide, 0.1 g of lead oxide, 22 g of cement and 9 g of carboxymethyl cellulose, and the evaluation was carried out according to the evaluation conditions of the example 1, the conversion of ethylbenzene was 76.35%; the selectivity of styrene was 95.5% and the yield of styrene was 72.91%.

This example is a comparative one to the example 5. In this example one rare-earth metal element was selected, while multiple rare-earth metal elements were selected for use in the example 5, through comparing the conversion of ethylbenzene, the selectivity and the yield of styrene between the two examples, it was found that the properties of the catalyst containing multiple rare-earth metal elements were obviously superior to that containing only one rare-earth metal element.

Example 10

According to the method of the example 1, the catalyst was prepared by using 275 g of iron oxide, 70 g of potassium carbonate, 3.8 g of praseodymium molybdenate, 4.1 g of lanthanum nitrate, 6 g of magnesium oxide, 0.5 g of cobalt nitrate, 1.0 g of copper oxide, 25 g of cement and 7 g of carboxymethyl cellulose, 100 ml of the catalyst particles of 3 mm in diameter were placed in the isothermal reactor having an inner diameter of 1 inch (about 25.4 mm), then the activity evaluation was performed under the conditions of: a reaction temperature of 600°C and the water ratio(water/diethylbenzene) of 4.0 (weight ratio), the evaluation results: the conversion of diethylbenzene 46.05%, the selectivity of divinylbenzene 91.50% and the yield of divinylbenzene 42.14%.

Example 11

The catalyst prepared according to the example 1 was evaluated according to the evaluation method of the example 1, except for the evaluation apparatus and conditions as following: a reaction pressure -0.02MPa, the liquid hourly space velocity 0.3 (liter ethylbenzene/liter catalyst • hr), a reaction temperature 580°C, the water ratio (water/ ethylbenzene) 1.0(weight ratio). The evaluation results: the conversion of ethylbenzene 56.90%, the selectivity of styrene 97.40% and the yield of styrene 55.42%.

Example 12

The catalyst prepared according to the example 3 was evaluated according to the evaluation method of the example 1, except for the conditions and apparatus of the evaluation as follows: a reaction pressure -0.05MPa, the liquid hourly space velocity 0.4 (liter ethylbenzene /liter catalyst • hr), a reaction temperature 630°C, the water ratio (water/ ethylbenzene) 1.3(weight ratio). The evaluation results: the conversion of ethylbenzene 80.80%, the selectivity of styrene 94.76% and the yield of styrene 76.57%.

Example 13

The catalyst prepared according to the example 3 was evaluated according to the evaluation method of the example 1, except for the conditions and apparatus of the evaluation as follows: a reaction pressure -0.07MPa, the liquid hourly space velocity 0.6 (liter ethylbenzene /liter catalyst • hr), a reaction temperature 650°C, the water ratio (water/ethylbenzene) 2.5(weight ratio). The evaluation results: the conversion of ethylbenzene 83.45%, the selectivity of styrene 93.76% and the yield of styrene 78.24%.

The examples show that: in the system of iron-potassium-molybdenum-magnesium-rare earth metal elements (at least two), the multiple metal oxides, a pore-forming agent and an adhesive are added in the catalysts of the invention, the resultant catalyst for the dehydrogenation, in combination with suitable technological conditions of the process, provides the suitability for use at a low water ratio(such as water ratio 1.3) and still having high conversion, selectivity and yield of products, and also exhibits very strong ability of self-regeneration, simple method for preparing the catalyst of the invention, short induction period of reaction and high flexibility for operation etc. as its advantages.

**Claims**

1. A dehydrogenation catalyst for producing unsaturated aromatics **characterized in that** said catalyst comprises $Fe_2O_3$ 40~70%, $K_2O$ 10~40%, $MoO_3$ 0~5%, MgO 0.05~5% and 0.001~5% of at least one oxide of the elements selected from the group consisting of Cu, Zn, Sc, Ti, W, Mn, Ni, Pd, Al, P, Bi, B, Sn, Pb and Si by weight, and that the catalyst comprises further at least two compounds of rare-earth metal elements, the content of which is from 2% to 15% on the basis of the oxides, and the rare-earth metal elements of the catalyst are selected from the group consisting of Ce, La, Pr, Nd and Sm.

2. The dehydrogenation catalyst according to claim 1 for producing unsaturated aromatics, **characterized in that** the content of MgO is from 0.5% to 4% by weight.

3. The dehydrogenation catalyst according to claim 1 for producing unsaturated aromatics, **characterized in that** the content of the at least one oxide of the elements selected from the group consisting of Cu, Zn, Sc, Ti, W, Mn, Ni, Pd, Al, P, Bi, B, Sn, Pb and Si is from 0.002% to 2% by weight percent.

4. The dehydrogenation catalyst according to claim 1 for producing unsaturated aromatics, **characterized in that** the content of the rare-earth metal elements in the catalyst is from about 3% to 8% based on the oxides.

5. The dehydrogenation catalyst according to claim 1 for producing unsaturated aromatics, **characterized in that** one rare-earth metal element is cerium.

6. The dehydrogenation catalyst according to claim 1 for producing unsaturated aromatics, **characterized in that** said catalyst comprises further a pore-forming agent and an adhesive.

7. A dehydrogenation process for producing unsaturated aromatics **characterized in that** said unsaturated aromatics are produced by contacting alkyl aromatics with said catalyst under the conditions of dehydrogenation, and the catalyst used comprises: $Fe_2O_3$ 40~70%, $K_2O$ 10~40%, $MoO_3$ 0~5%, MgO 0.05~5% and 0.001~5% of at least one oxide of the elements selected from the group consisting of Cu, Zn, Sc, Ti, W, Mn, Ni, Pd, Al, P, Bi, B, Sn, Pb and Si by weight percent, and further comprises at least two compounds of rare-earth metal elements, the content of which is from 2% to 15% on the basis of the oxides, and the rare-earth metal elements of the catalyst are selected from the group consisting of Ce, La, Pr, Nd and Sm.

8. The dehydrogenation process according to claim 7, **characterized in that** the reaction condition of said dehydrogenation includes the reaction temperature from 550 to 650°C, the reaction pressure from atmosphere to -0.07 MPa, the weight ratio of water to alkyl aromatics from 0.6 to 2.5, and the liquid hourly space velocity from 0.3 to 1.0 $hr^{-1}$.

9. The dehydrogenation process according to claim 7 or 8, **characterized in that** the reaction temperature is from 600°C to 630°.

10. The dehydrogenation process according to claim 7 or 8, **characterized in that** the reaction pressure is from -0.02 to -0.05 MPa.

11. The dehydrogenation process according to claim 7 or 8, **characterized in that** the weight ratio of water/alkyl aromatics is from 1.0 to 1.5.

12. The dehydrogenation process according to claim 7 or 8, **characterized in that** the liquid hourly space velocity is from 0.4 to 0.6 $hr^{-1}$.

13. The dehydrogenation process according to claim 7 or 8, **characterized in that** the content of the compounds of rare-earth metal elements in the catalyst is from about 3 % to 8 % based on the oxides.

14. The dehydrogenation process according to claim 7 or 8, **characterized in that** the content of MgO by weight percent is from 0.5 % to 4 %.

15. The dehydrogenation process according to claim 7 or 8, **characterized in that** the content of at least one oxide of the elements selected from the group consisting of Cu, Zn, Sc, Ti, W, Mn, Ni, Pd, Al, P, Bi, B, Sn, Pb, and Si is from 0.002 % to 2 % by weight percent.

16. The dehydrogenation process according to claim 7, **characterized in that** said catalyst comprises a pore-forming agent and an adhesive.

17. A use of said catalyst according to claim 1 in the preparation of styrene, divinylbenzene or methylstyrene.

**Revendications**

1. Catalyseur de déshydrogénation pour produire des substances aromatiques insaturées, **caractérisé en ce que**, ledit catalyseur comprend de 40 à 70% de $Fe_2O_3$, de 10 à 40% de $K_2O$, de 0 à 5% de $MoO_3$, de 0,05 à 5% de MgO et de 0,001 à 5% d'au moins un oxyde des éléments choisis dans le groupe formé par Cu, Zn, Sc, Ti, W, Mn, Ni, Pd, Al, P, Bi, B, Sn, Pb et Si, en poids, et **en ce que** le catalyseur comprend de plus au moins deux composés d'éléments métalliques des terres rares, dont la teneur est de 2% à 15% par rapport aux oxydes, et les éléments métalliques des terres rares du catalyseur sont choisis dans le groupe formé par Ce, La, Pr, Nd, et Sm.

2. Catalyseur de déshydrogénation selon la revendication 1, pour produire des substances aromatiques insaturées, **caractérisé en ce que** la teneur en MgO est de 0,5% à 4% en poids.

3. Catalyseur de déshydrogénation selon la revendication 1, pour produire des substances aromatiques insaturées, **caractérisé en ce que** la teneur en ledit au moins un oxyde des éléments choisis dans le groupe formé par Cu, Zn, Sc, Ti, W, Mn, Ni, Pd, Al, P, Bi, B, Sn, Pb et Si est de 0,002% à 2% en pourcent pondéral.

4. Catalyseur de déshydrogénation selon la revendication 1, pour produire des substances aromatiques insaturées, **caractérisé en ce que** la teneur en éléments métalliques des terres rares dans le catalyseur est d'environ 3% à 8% par rapport aux oxydes.

5. Catalyseur de déshydrogénation selon la revendication 1, pour produire des substances aromatiques insaturées, **caractérisé en ce qu'**un élément métallique des terres rares est le cérium.

6. Catalyseur de déshydrogénation selon la revendication 1, pour produire des substances aromatiques insaturées, **caractérisé en ce que** ledit catalyseur comprend de plus un agent porogène et un adhésif.

7. Procédé de déshydrogénation pour produire des substances aromatiques insaturées, **caractérisé en ce que**, lesdites substances aromatiques insaturées sont produites en mettant des substances alkylaromatiques en contact avec ledit catalyseur dans des conditions de déshydrogénation, et le catalyseur utilisé comprend : 40 à 70% de $Fe_2O_3$, de 10 à 40% de $K_2O$, de 0 à 5% de $MoO_3$, de 0,05 à 5% de MgO et de 0,001 à 5% d'au moins un oxyde des éléments choisis dans le groupe formé par Cu, Zn, Sc, Ti, W, Mn, Ni, Pd, Al, P, Bi, B, Sn, Pb et Si, en pourcent pondéral, et comprend de plus au moins deux composés d'éléments métalliques des terres rares, dont la teneur est de 2% à 15% par rapport aux oxydes, et les éléments métalliques des terres rares du catalyseur sont choisis dans le groupe formé par Ce, La, Pr, Nd et Sm.

8. Procédé de déshydrogénation selon la revendication 7, **caractérisé en ce que** la condition de réaction de ladite déshydrogénation inclut la température de réaction de 550 à 650°C, la pression de réaction de la pression atmosphérique jusqu'à -0,07 MPa, le rapport pondéral de l'eau aux substances alkylaromatiques de 0,6 à 2,5, et la vitesse spatiale horaire du liquide de 0,3 à 1,0 $h^{-1}$.

9. Procédé de déshydrogénation selon la revendication 7 ou 8, **caractérisé en ce que** la température de réaction est de 600°C à 630°C.

**10.** Procédé de déshydrogénation selon la revendication 7 ou 8, **caractérisé en ce que** la pression de réaction est de -0,02 à -0,05 Mpa.

**11.** Procédé de déshydrogénation selon la revendication 7 ou 8, **caractérisé en ce que** le rapport pondéral eau/ substances alkylaromatiques est de 1,0 à 1,5.

**12.** Procédé de déshydrogénation selon la revendication 7 ou 8, **caractérisé en ce que** la vitesse spatiale horaire du liquide est de 0,4 à 0,6 h$^{-1}$.

**13.** Procédé de déshydrogénation selon la revendication 7 ou 8, **caractérisé en ce que**, la teneur en les composés des éléments métalliques des terres rares dans le catalyseur est d'environ 3% à 8% par rapport aux oxydes.

**14.** Procédé de déshydrogénation selon la revendication 7 ou 8, **caractérisé en ce que**, la teneur en MgO est de 0,5% à 4% en pourcentage pondéral.

**15.** Procédé de déshydrogénation selon la revendication 7 ou 8, **caractérisé en ce que** la teneur en au moins un oxyde des éléments choisis dans le groupe formé par Cu, Zn, Sc, Ti, W, Mn, Ni, Pd, Al, P, Bi, B, Sn, Pb, et Si est de 0,002% à 2% en pourcentage pondéral.

**16.** Procédé de déshydrogénation selon la revendication 7, **caractérisé en ce que** ledit catalyseur comprend un agent porogène et un adhésif.

**17.** Utilisation dudit catalyseur selon la revendication 1, pour la préparation du styrène, du divinylbenzène ou du méthylstyrène.


**Patentansprüche**

**1.** Dehydrierungskatalysator zum Herstellen von ungesättigten Aromaten, **dadurch gekennzeichnet, daß** der Katalysator $Fe_2O_3$ 40 ~ 70 Gew.-%, $K_2O$ 10 ~ 40 Gew.-%, $MoO_3$ 0 ~ 5 Gew.-%, MgO 0,05 ~ 5 Gew.-% und 0,001 ~ 5 Gew.-% wenigstens eines Oxids der Elemente, die ausgewählt sind aus der Gruppe bestehend aus Cu, Zn, Sc, Ti, W, Mn, Ni, Pd, Al, P, Bi, B, Sn, Pb und Si, umfaßt, und daß der Katalysator weiter wenigstens zwei Verbindungen von Seltenerdmetallelementen umfaßt, deren Gehalt von 2 % bis 15 % auf der Basis der Oxide ist, und wobei die Seltenerdmetallelemente des Katalysators ausgewählt sind aus der Gruppe bestehend aus Ce, La, Pr, Nd und Sm.

**2.** Dehydrierungskatalysator nach Anspruch 1 zum Herstellen ungesättigter Aromaten, **dadurch gekennzeichnet, daß** der Gehalt an MgO von 0,5 Gew.-% bis 4 Gew.-% ist.

**3.** Dehydrierungskatalysator nach Anspruch 1 zum Herstellen ungesättigter Aromaten, **dadurch gekennzeichnet, daß** der Gehalt des wenigstens einen Oxids der Elemente, die ausgewählt sind aus der Gruppe bestehend aus Cu, Zn, Sc, Ti, W, Mn, Ni, Pd, Al, P, Bi, B, Sn, Pb und Si von 0,002 Gew.-% bis 2 Gew.-% ist.

**4.** Dehydrierungskatalysator nach Anspruch 1 zum Herstellen ungesättigter Aromaten, **dadurch gekennzeichnet, daß** der Gehalt an den Seltenerdmetallelementen in dem Katalysator von etwa 3 % bis 8 % auf der Basis der Oxide ist.

**5.** Dehydrierungskatalysator nach Anspruch 1 zum Herstellen ungesättigter Aromaten, **dadurch gekennzeichnet, daß** ein Seltenerdmetallelement Cer ist.

**6.** Dehydrierungskatalysator nach Anspruch 1 zum Herstellen ungesättigter Aromaten, **dadurch gekennzeichnet, daß** der Katalysator weiter ein porenbildendes Agens und einen Klebstoff umfaßt.

**7.** Dehydrierungsverfahren zum Herstellen ungesättigter Aromaten, **dadurch gekennzeichnet, daß** die ungesättigten Aromaten hergestellt werden durch Kontaktieren von Alkylaromaten mit dem Katalysator unter den Bedingungen einer Dehydrierung, und wobei der verwendete Katalysator umfaßt: $Fe_2O_3$ 40 ~ 70 Gew.-%, $K_2O$ 10 ~ 40 Gew.-%, $MoO_3$ 0 ~ 5 Gew.-%, MgO 0,05 ~ 5 Gew.-% und 0,001 ~ 5 Gew.-% wenigstens eines Oxids der Elemente, die ausgewählt werden aus der Gruppe bestehend aus Cu, Zn, Sc, Ti, W, Mn, Ni, Pd, Al, P, Bi, B, Sn, Pb und Si, und welcher weiter umfaßt wenigstens zwei Verbindungen von Seltenderdmetallementen, deren Gehalt von 2 % bis 15 % auf der Basis der Oxide ist, und wobei die Seltenerdmetallelemente des Katalysators ausgewählt werden aus der Gruppe bestehend aus Ce, La, Pr, Nd und Sm.

8. Dehydrierungsverfahren nach Anspruch 7, **dadurch gekennzeichnet, daß** die Reaktionsbedingung der Dehydrierung die Reaktionstemperatur von 550 bis 650°C, den Reaktionsdruck von Atmosphärendruck bis -0,07 MPa, das Gewichtsverhältnis von Wasser zu Alkylaromaten von 0,6 bis 2,5 und die Flüssigkeitsstundenraumgeschwindigkeit von 0,3 bis 1,0 hr$^{-1}$ einschließt.

9. Dehydrierungsverfahren nach Anspruch 7 oder 8, **dadurch gekennzeichnet, daß** die Reaktionstemperatur von 600°C bis 630°C beträgt.

10. Dehydrierungsverfahren nach Anspruch 7 oder 8, **dadurch gekennzeichnet, daß** der Reaktionsdruck von -0,02 bis -0,05 MPa ist.

11. Dehydrierungsverfahren nach Anspruch 7 oder 8, **dadurch gekennzeichnet, daß** das Gewichtsverhältnis von Wasser/Alkylaromaten von 1,0 bis 1,5 ist.

12. Dehydrierungsverfahren nach Anspruch 7 oder 8, **dadurch gekennzeichnet, daß** die Flüssigkeitsstundenraumgeschwindigkeit von 0,4 bis 0,6 hr$^{-1}$ ist.

13. Dehydrierungsverfahren nach Anspruch 7 oder 8, **dadurch gekennzeichnet, daß** der Gehalt der Verbindungen der Seltenerdmetallelemente in dem Katalysator von etwa 3 % bis 8 % auf der Basis der Oxide ist.

14. Dehydrierungsverfahren nach Anspruch 7 oder 8, **dadurch gekennzeichnet, daß** der Gehalt an MgO von 0,5 Gew.-% bis 4 Gew.-% ist.

15. Dehydrierungsverfahren nach Anspruch 7 oder 8, **dadurch gekennzeichnet, daß** der Gehalt wenigstens eines Oxids der Elemente, die ausgewählt werden aus der Gruppe bestehend aus Cu, Zn, Sc, Ti, W, Mn, Ni, Pd, Al, P, Bi, B, Sn, Pb und Si von 0,002 Gew.-% bis 2 Gew.-% ist.

16. Dehydrierungsverfahren nach Anspruch 7, **dadurch gekennzeichnet, daß** der Katalysator ein porenbildendes Agens und einen Klebstoff umfaßt.

17. Verwendung des Katalysators nach Anspruch 1 bei der Herstellung von Styrol, Divinylbenzol oder Methylstyrol.